# EUROPÄISCHE PATENTSCHRIFT

(11) **EP 0 573 910 B1**
(45) Veröffentlichungstag und Bekanntmachung des Hinweises auf die Patenterteilung: **21.01.1998**
(21) Anmeldenummer: 93108993.2
(22) Anmeldetag: 04.06.1993
(51) Int. Cl.: C07C 209/36, B01J 23/89

(54) **Verwendung eines Katalysator für die selektive Hydrierung von Halogennitroaromaten zu aromatischen Halogenaminen**
Use of a catalyst for selectively hydrogenating halonitroaromatics into aromatic haloamines
Utilisation d'un catalyseur d'hydrogénation sélectives des composés aromatiques nitrés et halogénées en amines halogénées aromatique

(30) Priorität: 09.06.1992 DE 4218866
(43) Veröffentlichungstag der Anmeldung: 15.12.1993
(73) Patentinhaber: Degussa Aktiengesellschaft, 60311 Frankfurt (DE)
(72) Erfinder: Deller, Klaus, Dr., D-6452 Hainburg (DE); Despeyroux, Bertrand, Dr., D-6450 Hanau 1 (DE); Peldszus, Erik, D-6467 Hasselroth 1 (DE); Kleinwächter, Beate, Dr., D-6450 Hanau 9 (DE)

(56) Entgegenhaltungen:
- EP-A- 0 027 979
- EP-A- 0 496 446
- GB-A- 2 052 294
- US-A- 3 546 297
- US-A- 3 928 451

## Beschreibung

Die Erfindung betrifft die Verwendung eines durch Kupfer modifizierten Platinkatalysators auf einem Träger aus Aktivkohle für die Hydrierung von Halogennitroaromaten zu aromatischen Halogenaminen mit guter Aktivität, Selektivität und Ausbeute.

Aromatische Halogenamine sind wichtige Ausgangsstoffe für die Produktion von Pharmazeutika, Farbstoffen, Pestiziden und Herbiziden. Sie werden zum Beispiel durch katalytische Hydrierung aus den entsprechenden Halogennitroaromaten hergestellt. Die Hydrierung erfolgt gewöhnlich unter Verwendung eines Lösungsmittels zur Überführung der festen Halogennitroaromaten in die flüssige Phase. Typische Hydrierbedingungen sind Reaktionstemperaturen von 25 bis 250°C und Wasserstoffdrücke von 0.1-35 MPa (1 bis 350 bar).

Bei der katalytischen Hydrierung tritt das Problem auf, daß nicht nur die Nitrogruppe zur Amingruppe reduziert wird, sondern auch die Halogenatome durch Wasserstoffatome substituiert werden, wobei die entsprechende Halogensäure gebildet wird. Die gebildete Säure führt zu Korrosionen im Reaktor und muß deshalb durch Einsatz eines möglichst selektiven Katalysators, der nur die Nitrogruppe hydriert, vermieden werden.

In der Vergangenheit wurde versucht, die Dehalogenierung durch Einsatz von Edelmetallträgerkatalysatoren zu vermeiden, die zur Verbesserung der Selektivität noch durch geeignete Zusätze modifiziert wurden.

Als geeignete Träger sind Ruß, insbesondere Acetylenruß und Aktivkohle bekannt geworden. Als Edelmetallkomponente wird überwiegend Platin eingesetzt. Palladium weist zwar eine bessere Aktivität als Platin auf, besitzt jedoch auch eine geringere Selektivität. Die Selektivität kann durch Modifizierung des Katalysators mit Schwefel oder Schwermetallen verbessert werden.

Salek et al. (A. Salek, J.M. Berak, S. Tobola, W. Ormaniec, A. Teichert: Przemysl Chemiczny 58 (1979) 8; 425 - 427) berichten über Untersuchungen zur Verbesserung der Aktivität und Selektivität eines Pt/Acetylenruß-Katalysators bei der Hydrierung von 3,4-Dichlornitrobenzol. Sie kommen zu dem Ergebnis, daß eine gute Aktivität und Selektivität erzielt werden kann, wenn der Platingehalt des Katalysators, bezogen auf das Gewicht des Trägers, mindestens 5 Gew.-% beträgt, zu dem Reaktionsgemisch 15 Gew.-% Anilin zugegeben werden und der Katalysator noch durch 0,25 bis 1 % Kupfer (bei gleichzeitiger Ausfällung von Platin und Kupfer) modifiziert wird. Die Modifizierung des Katalysators mit Kupfer bewirkt mit steigendem Kupfergehalt eine zuerst schnelle Verminderung der Chloridkonzentration in der wäßrigen Phase und eine nur langsame Verschlechterung der Aktivität. Bei 0,25 Gew.-% Kupfer liegt der Chloridgehalt noch bei über 2 Gew.-% und erst oberhalb von 0,5 Gew.-% Kupfer sinkt der Chloridgehalt unter 0,5 Gew.-%. Die Modifizierung des Katalysators mit Eisen verschlechtert die Selektivität.

Salek et al. machen keine genauen Angaben über die Herstellung ihrer Katalysatoren. Die Niederschlagung der Metallkomponenten auf dem Rußträger erfolgte durch Ausfällen der Metallhydroxide aus einer Lösung der Metallsalze mit einer NaHCO₃-Lösung. Es werden keine Angaben zu der Temperatur bei diesem Prozeß gemacht. Die Katalysatoren wurden offensichtlich ohne vorherige Reduktion für die Hydrierung eingesetzt.

Allgemein ist eine industrielle Verwendung von Katalysatoren auf Basis von Rußträgern wegen Filtrationsproblemen während der naßchemischen Katalysatorherstellung bzw. während des Einsatzes im Verfahren ungeeignet.

In der DE-OS 20 42 368 werden mit Blei, Wismut oder Silber modifizierte Pt/C-Katalysatoren beschrieben. Als optimaler Platin-Gehalt des Katalysators werden 5 Gew.-% angegeben. Nicht modifizierte Katalysatoren führen bei der Hydrierung von 2,5 Dichlornitrobenzol zu vollständiger Abspaltung des Chlors. Eine Modifizierung mit 5 Gew.-% Kupfer ergab im Gegensatz zu den Arbeiten von Salek et al. immer noch eine Chlorabspaltung von 75 %, was wahrscheinlich darauf zurückzuführen ist, daß in der DE-OS 20 42 368 kein Anilin zur Verhinderung der Chlorabspaltung eingesetzt wird. Erst die Zugabe von 5 Gew.-% Blei, Wismut oder Silber verringerte die Chlorabspaltung unter 1 %.

Die britische Patentanmeldung GB 2 024 643 beschreibt einen Platin-Katalysator auf Acetylenruß, der zur Verbesserung der Aktivität und zur Verminderung der Dehalogenierung und der Hydroxylamin-Bildung mit Eisen modifiziert ist. Die optimale Beladung des Trägermaterials mit Platin liegt bei 5 Gew.-%, und für das Mol-Verhältnis von Eisen zu Platin wird ein Bereich von 2 : 1 bis 16 : 1 angegeben, was einem Gewichtsverhältnis von etwa 1 : 2 bis 4 : 1 entspricht.

Von den Rußträgern wird in der britischen Patentanmeldung eine geringe Porosität und daraus folgend eine relativ kleine spezifische Oberfläche von weniger als 300 m²/g verlangt. In den Beispielen wird ein Rußträger mit nur 35 m²/g eingesetzt. 70 % der Teilchen dieses Trägers haben eine Korngröße unter 1 µm. Eine gute Aktivität des Katalysators wird gemäß der Anmeldung durch einen optimalen, feinteiligen Niederschlag der katalytisch aktiven Komponenten Platin und Eisen auf den feinkörnigen, nicht porösen Rußteilchen erzielt. Dazu ist es erforderlich, Platin und Eisen zunächst als Oxide, Hydroxide oderCarbonate bei einer Temperatur von ca. 90° C in einer Suspension von Ruß aus einer wäßrigen Lösung der Salze der Metallkomponenten auszufällen und anschließend bei Raumtemperatur mit Formaldehyd zu reduzieren.

Für die optimale Verteilung des Platins und Eisen auf den Rußteilchen ist es von ausschlaggebender Bedeutung, daß die Reduktion bei Raumtemperatur durchgeführt wird. Eine Reduktion bei 90° C liefert wesentlich schlechtere Ergebnisse.

In der britischen Patentanmeldung werden auch Vergleichsversuche mit höheroberflächigen Trägermaterialien beschrieben. Zum Einsatz kamen eine Aktivkohle (Träger von Control F) mit einer spezifischen Oberfläche von 500 m²/g und ein Rußträger (Träger von Control G) mit 1050 m²/g. 70 % der Teilchen der Aktivkohle hatten eine Korngröße unter 30 µm, während 70 % der Teilchen des Rußträgers größer als 10 µm waren. Der Katalysator auf Aktivkohle besaß eine geringe Aktivität und führte zu einer Hydrierzeit von 133 Minuten gegenüber 84 Minuten bei Einsatz des niederoberflächigen Rußträgers.

Die Hydrierung mit dem hochoberflächigen Rußträger mußte sogar nach 130 Minuten wegen zu starker Säurebildung abgebrochen werden.

Die Veröffentlichungen "Platin Katalysatoren für die Hydrierung von Nitrobenzolen" von J. Strutz und E. Hopf in Chem. Ing. Tech. 60 (1988) 4, 297 - 298 und "Selektive Hydrierung mit neuen Platinkatalysatoren" von J.B.F. Anderson, K.G. Griffin und R.E. Richards in Chemie-Technik, 18 (1989), 5, 40 - 44 beschreiben reine Platin-Hydrierkatalysatoren auf Aktivkohleträgern.
J.B.F. Anderson et al. erreichen mit ihrem Katalysator bei 1 %iger Beladung mit Platin gute Selektivitäten, geben jedoch nicht die dabei erzielten Ausbeuten an. J. Strutz und E. Hopf berichten ebenfalls über Katalysatoren mit 1 %iger Platin-Beladung. Die von ihnen erzielten Ausbeuten sind jedoch ungenügend.

Die US-Patentschriften 3,928,451 und 3,546,297 offenbaren die Verwendung von Platin auf Aktivkohle beziehungsweise von Platin, Nickel und Chrom auf Aktivkohle für die selektive Hydrierung von Halogennitroaromaten.

Die nicht vorveröffentlichte europäische Offenlegungsschrift EP 0 496 446 A1 beschreibt ein Verfahren für die Herstellung von Chlortrifluorethen unter Verwendung eines Katalysators, welcher Kupfer und ein Metall der Gruppe IIIV des Periodensystems im Gewichtsverhältnis von 0,1 bis 20 auf Aktivkohle enthält.

Die vorgestellten Katalysatoren nach dem Stand der Technik für die Hydrierung von Halogennitroaromaten zu aromatischen Halogenaminen sind unbefriedigend. Sie arbeiten zum Teil mit einem sehr hohen Edelmetalleinsatz, und ihre Selektivität und Ausbeute sind nicht ausreichend.

Es ist daher Aufgabe der Erfindung, einen Katalysator anzugeben, welcher sich bei der Verwendung für die Hydrierung von Halogennitroaromaten zu aromatischen Halogenaminen durch eine gute Selektivität und Langzeitstabilität auszeichnet und darüber hinaus nur einen geringen Edelmetallbedarf aufweist.

Es wurde gefunden, daß ein Katalysator aus Platin und Kupfer auf einem Träger aus Aktivkohle diese Anforderungen erfüllt, wenn er, bezogen auf den Aktivkohleträger, 0,1 bis 5,0, vorzugsweise 0,5 bis 3,5, Gew.-% Platin enthält und ein Gewichtsverhältnis zwischen Platin und Kupfer von 5 : 1 bis 20 : 1, vorzugsweise 10 : 1, aufweist.

Der Katalysator besitzt besonders günstige Eigenschaften, wenn der mittlere Teilchendurchmesser der Aktivkohle 15 bis 30 µm beträgt, ihre spezifische Oberfläche größer als 500 m²/g und ihr Gesamtporenvolumen größer als 0,5 ml/g sind.

Der Katalysator ist erhältlich durch Imprägnieren des in Wasser suspendierten Trägermaterials mit Verbindungen des Platins und Kupfers, durch Ausfällen dieser Verbindungen aus wäßrigen Lösungen ihrer leicht löslichen Verbindungen, Reduzieren, Filtrieren und Waschen des fertigen Katalysators, wobei die wäßrige Suspension 5 bis 30 Gew.-% Aktivkohle als Katalysatorträger enthält, und Platin und Kupfer der Suspension in Form einer Lösung von wasserlöslichen Platinverbindungen, vorzugsweise Hexachloroplatinsäure, und eines wasserlöslichen Kupfersalzes, vorzugsweise Kupferacetat, zugegeben werden, wobei der Gehalt der Lösung an Platinmetall und Kupfermetall entsprechend der gewünschten Beladung der eingesetzten Menge Aktivkohle bemessen ist und die Suspension zusammen mit der Lösung aus den wasserlöslichen Platin- und Kupferverbindungen unter Rühren auf 70 - 100°C erwärmt wird, bevor Platin und Kupfer gleichzeitig durch Zugabe einer Base wie zum Beispiel Natriumcarbonat oder Natronlauge in Form ihrer schwer löslichen Verbindungen auf der Aktivkohle niedergeschlagen und anschließend bei unveränderter Temperatur durch Zugabe eines Reduktionsmittels wie Hydrazin, Natriumformiat, Natriumboranat oder Formaldehyd, vorzugsweise Formaldehyd, reduziert werden.

Der erfindungsgemäß zu verwendende Katalysator weist bessere Hydrieraktivitäten wie bisher verwendete Katalysatoren auf, zeichnet sich jedoch durch einen geringen Edelmetalleinsatz von nur 0,1 bis 5,0, vorzugsweise 0,5 bis 3,5, Gew.-% aus. Die Selektivität des Katalysators ist gegenüber dem Stand der Technik durch Zugabe einer geringen Menge Kupfer verbessert. Das Gewichtsverhältnis von Platin zu Kupfer kann zwischen 5 : 1 bis 20 : 1 betragen, vorzugsweise liegt es bei 10 : 1. Bei einer Beladung des Trägers mit 1 Gew.-% Platin entspricht das 0,1 Gew.-% Kupfer.

Die hervorragenden Eigenschaften des erfindungsgemäß zu verwendenden Katalysators bei der selektiven Hydrierung von Halogennitroaromaten beruhen auf der Kombination eines Aktivkohleträgers mit einer geeigneten Beladung mit Platin und Kupfer. Ein geeigneter Aktivkohleträger ist in der DE-PS 38 23 301 beschrieben. Er weist eine BET-Oberfläche > 500 m²/g, ein Gesamtporenvolumen > 0,5 ml/g und einen Aschegehalt < 5 Gew.-% auf. Seine mittlere Teilchengröße beträgt 15 bis 30 µm.

Das Herstellverfahren verwendet wasserlösliche Platinverbindungen, vorzugsweise Hexachloroplatinsäure, und Kupfersalze wie Kupferacetat, Kupferchlorid, Kupfernitrat und Kupfersulfat. Besonders günstig ist die Verwendung von Kupferacetat. Platin und Kupfer werden aus der Lösung durch Zugabe einer Base als Hydroxidverbindungen ausgefällt und auf dem Träger niedergeschlagen. Wichtig für die optimale Metalldispersion über das ganze Korn der Aktivkohle ist, daß die Ausfällung und auch die anschließende Reduktion der Hydroxidverbindungen in einem Temperaturbereich von 70 bis 100°C durchgeführt werden. In diesem erhöhten Temperaturbereich wird ein Katalysator gebildet, bei dem Platin und Kupfer in fein verteilter Form reduziert auf Aktivkohle vorliegen.

Die Imprägnierung der Aktivkohle mit den Metallverbindungen kann entweder wie oben beschrieben durch gleichzeitige Ausfällung beider Metallkomponenten aus der Lösung erfolgen oder nacheinander. Bei getrennter Ausfällung wird die Aktivkohle zunächst mit Platin und in einem zweiten Prozeßschritt mit Kupfer imprägniert.

Die Erfindung wird nun anhand einiger Beispiele erläutert. Beispiel 1 beschreibt die Herstellung von erfindungsgemäß zu verwendenden Katalysatoren und Vergleichskatalysatoren aus dem Stand der Technik. Die Katalysatoren wurden in den Beispielen 2 bis 4 in einem adiabatischen Hochdrucktest, einem isothermen Hochdrucktest und in einem Niederdrucktest auf Aktivität und Selektivität hin untersucht und miteinander verglichen. In Beispiel 5 wurden Mehrzyklen-Hydrierungen mit einem mit Schwefel modifizierten Standardkatalysator und mit einem erfindungsgemäß zu verwendenden Katalysator zum Vergleich ihrer Langzeitstabilität durchgeführt. Die Ergebnisse der Beispiele sind in den Tabellen 1 bis 5 niedergelegt und in der einzigen Figur dargestellt.

### Beispiel 1: Katalysatorherstellung

Für alle Katalysatoren wurde als Träger Aktivkohle mit folgenden Materialdaten eingesetzt:

### Material Aktivkohle

| | |
|---|---|
| Spez. Oberfläche BET | 1.000 m²/g (ASTM-D-3663) |
| Gesamtporenvolumen | 1,01 ml/g (ASTM-D-4284) |
| Schüttgewicht | 210 g/l |
| Mittlere Korngröße | 24,0 µm (ASTM-D-4464) |

### A) Herstellung der erfindungsgemäß zu verwendenden Katalysatoren

Zur Herstellung eines erfindungsgemäß zu verwendenden Katalysators mit 1 % Platin-Beladung und 0,1 Gew.-% Kupfer wurden 100 g Aktivkohle in destilliertes Wasser bei einer Rührgeschwindigkeit von 300 U/min eingerührt. In diese Suspension wurden wäßrige Lösungen von 2,5 g Hexachloroplatinsäure-hexahydrat -hexahydrat (entspricht 1 g Pt) und 0,316 g Kupferacetatmonohydrat (entspricht 0,1 g Cu) gegeben, und anschließend wurde die Suspension auf 80° C erwärmt. Dann wurden zur Ausfällung der Hydroxide Natronlauge unter ständigem Rühren zugefügt. Zur Reduktion der Metallverbindungen wurden schließlich 0,3 ml 37 %ige Formaldehydlösung zugegeben. Auch während der Reduktion wurde die Temperatur der Suspension auf 80° C konstant gehalten. Nach der Reduktion wurde der Katalysator über eine Nutsche abfiltriert und mit destilliertem Wasser gewaschen. Der Katalysator wurde im nassen Zustand für die Hydrierung eingesetzt. Erfindungsgemäße Katalysatoren mit abweichenden Platin- und Kupfer-Beladungen wurden durch entsprechende Vervlelfachung der Mengen-Hexachloroplatinsäure, Kupferacetat und Natronlauge hergestellt.

### B) Herstellung von Vergleichskatalysatoren

Zum Vergleich mit den erfindungsgemäß zu verwendenden Katalysatoren wurden reine Platin/Aktivkohle- und durch Schwefel modifizierte Platin/Aktivkohle-Katalysatoren hergestellt. Die Herstellung erfolgte sinngemäß wie unter A) beschrieben. Es wurde jedoch kein Kupferacetat zugegeben. Die Modifizierung mit Schwefel wurde nach dem in der DE-PS 21 50 220 beschriebenen Verfahren durchgeführt.

Tabelle 1 listet alle für die Vergleichsversuche der Beispiele 2 bis 5 hergestellten Katalysatoren auf. Die erfindungsgemäß zu verwendenden Katalysatoren sind mit dem Buchstaben K und einer fortlaufenden Numerierung gekennzeichnet. Die Katalysatoren K 1 bis K 4 unterscheiden sich durch unterschiedlich starke Beladungen des Katalysatorträgers mit Platin. Das Gewichtsverhältnis zwischen Platin und Kupfer beträgt jeweils 10 : 1. Die Vergleichskatalysatoren VK 1.1, VK 3.1, VK4.1 aus dem Stand der Technik entsprechen den Katalysatoren K 1, K 3 und K 4, sind jedoch nicht mit Kupfer modifiziert. Die Vergleichskatalysatoren VK 1.2, VK 3.2 und VK 4.2 sind dagegen gemäß dem Stand der Technik mit Schwefel modifiziert (sulfidiert).

Zur Testung des Einflusses des Gewichtsverhältnisses von Platin zu Kupfer wurden die Katalysatoren K 5 bis K 11 mit unterschiedlichen Kupfer-Beladungen von 0,01 % bis 0,15 % bei gleichbleibender Platin-Beladung von 1 % hergestellt.

**Tabelle 1 :**

| Katalysatoren | | | | |
|---|---|---|---|---|
| Katalysator | | Beladung | | |
| | | Pt | Cu | S |
| Katalysator | K 1 | 1 % | 0,1 % | - |
| Katalysator | K 2 | 2 % | 0,2 % | - |
| Katalysator | K 3 | 3 % | 0,3 % | - |
| Katalysator | K 4 | 5 % | 0,5 % | - |
| Vergleichskatalysator | VK 1.1 | 1 % | - | - |
| Vergleichskatalysator | VK 3.1 | 3 % | - | - |
| Vergleichskatalysator | VK 4.1 | 5 % | - | - |
| Vergleichskatalysator | VK 1.2 | 1 % | - | sulfidiert |
| Vergleichskatalysator | VK 3.2 | 3 % | - | sulfidiert |
| Vergleichskatalysator | VK 4.2 | 5 % | - | sulfidiert |
| Katalysator | K 5 | 1 % | 0,01 % | - |
| Katalysator | K 6 | 1 % | 0,05 % | - |
| Katalysator | K 7 | 1 % | 0,06 % | - |
| Katalysator | K 8 | 1 % | 0,07 % | - |
| Katalysator | K 9 | 1 % | 0,08 % | - |
| Katalysator | K 10 | 1 % | 0,10 % | - |
| Katalysator | K 11 | 1 % | 0,15 % | - |

### Beispiel 2: Adiabatische Hochdruck-Hydrierungen von 3,4-Dichlornitrobenzol mit verschiedenen Katalysatoren

Die Hydrierungen wurden in einem 500 ml Hastelloy-Autoklaven durchgeführt. Zum Einsatz kamen 75 g 3,4-Dichlornitrobenzol, gelöst in 125 ml Toluol und 0,375 g Katalysator (Trockenmasse) entsprechend 0,5 Gew.-% Katalysator bezogen auf Dichlornitrobenzol.

Die Katalysatormenge von 0,375 g Trockenmasse wurde im Falle der mit 1 % Platin beladenen Katalysatoren eingesetzt. Bei den mit 3 % bzw. 5 % Platin beladenen Katalysatoren wurde jeweils nur 1/3 bzw. 1/5 der obigen Menge verwendet.

**Tabelle 2 :**

| Adiabatische Hochdruckhydrierung von 3,4-Dichlornitrobenzol | | | | | | | |
|---|---|---|---|---|---|---|---|
| Katalysator | Beladung | | t [min] | Tmax [°] | Gaschromatografie | | |
| | Pt [Gew.-%] | Cu [Gew.-%] | | | DCA | DCNB | Dehalo |
| K 1 | 1 | 0,1 | 6,0 | 106,0 | 100 | 0 | 0 |
| K 3 | 3 | 0,3 | 14,5 | 78,0 | 100 | 0 | 0 |
| K 4 | 5 | 0,5 | 30,0 | 62,0 | 100 | 0 | 0 |
| VK 1.1 | 1 | - | 3,0 | 127,0 | 99,1 | 0 | 0,9 |
| VK 3.1 | 3 | - | 7,0 | 89,0 | 99,4 | 0 | 0,6 |
| VK 4.1 | 5 | - | 13,0 | 78,5 | 99,5 | 0 | 0,5 |
| VK 1.2 | 1 | sulfidiert | 6,0 | 100,0 | 100 | 0 | 0 |
| VK 3.2 | 3 | sulfidiert | 20,5 | 71,0 | 100 | 0 | 0 |
| VK 4.2 | 5 | sulfidiert | 34,5 | 60,5 | 100 | 0 | 0 |
| DCNB = 3,4-Dichlornitrobenzol (Edukt) | | | | | | | |
| DCA = 3,4-Dichloranilin (Produkt) | | | | | | | |
| Dehalo = Dehalogenierungsprodukte | | | | | | | |
| t = Reaktionszeit bis Tmax erreicht ist | | | | | | | |
| Tmax = maximale Temperatur | | | | | | | |

Der Wasserstoffdruck betrug bei den Hydrierversuchen 60 bar, die Starttemperatur 40° C. Während der Hydrierung wurde das Reaktionsgemisch ständig gerührt, die Temperatur und der Wasserstoffverbrauch gemessen.

Die Hydrierung von Dichlornitrobenzol ist exotherm. Durch die frei werdende Wärmemenge stieg daher die Temperatur des Reaktionsgemisches innerhalb weniger Minuten auf einen Maximalwert an und kühlte sich dann langsam ab.

Die Zeit bis zum Erreichen der maximalen Temperatur ist als "Reaktionszeit" t in Tabelle 2 zusammen mit der maximalen Temperatur Tmax aufgeführt. Nach jeweils einer Stunde wurden die Reaktionsgemische gaschromatografisch untersucht.

Tabelle 2 zeigt, daß bei allen Versuchen nach einer Stunde Reaktionszeit im Reaktionsgemisch kein als Edukt eingesetztes 3,4-Dichlornitrobenzol (DCNB) mehr nachweisbar war. Bei den mit Kupfer modifizierten Katalysatoren K 1, K 3 und K 4 sind keinerlei Dehalogenierungsprodukte (Dehalo) nachweisbar, während die nicht modifizierten Vergleichskatalysatoren VK 1.1, VK 3.1 und VK 4.1 zwar eine höhere Aktivität besitzen, aber Dehalogenierungsprodukte erzeugen und somit eine schlechtere Selektivität aufweisen als die erfindungsgemäßen Katalysatoren.

Die mit Schwefel modifizierten Vergleichskatalysatoren weisen unter den angegebenen Versuchsbedingungen ähnliche Aktivitäten wie die erfindungsgemäß zu verwendenden Katalysatoren auf und erzeugen ebenfalls keine Dehalogenierungsprodukte. Wie Beispiel 5 zeigen wird, ist aber ihre Langzeitstabilität wesentlich schlechter als die der erfindungsgemäß zu verwendenden Katalysatoren.

### Beispiel 3: Isotherme Hochdruckhydrierung von 3,4-Dichlornitrobenzol

Die isothermen Hochdruckhydrierungen wurden in einem Autoklaven aus V4A-Stahl mit 1 1 Fassungsvermögen durchgeführt. Zum Einsatz kamen die erfindungsgemäßen Katalysatoren K 1, K 2 und K 3 und der Vergleichskatalysator VK 1.2, ein mit Schwefel modifizierter Standardkatalysator.

Für die isothermen Hydrierungen wurden folgende Versuchsbedingungen gewählt:

| | |
|---|---|
| Einwaage | 100 g 3,4-DCNB (3,4-Dichlornitrobenzol) |
| | 520 g Toluol |
| | 0,2 % Katalysator-Trockenmasse bezogen auf DCNB |
| Temperatur | T = 100° C |
| H₂-Druck | p = 60 bar |
| Rührerdrehzahl | n = 1.150 U/min |

Der Gehalt der Reaktionsgemische an dem gewünschten Produkt 3,4-Dichloranilin (3,4-DCA) wurde in regelmäßigen Abständen geprüft. Die Ergebnisse sind in Tabelle 3 zusammengefaßt. Alle Katalysatoren wiesen eine 100 %ige Selektivität auf, d. h. nach vollständiger Hydrierung konnten keine Dehalogenlerungsprodukte nachgewiesen werden. Die mit Kupfer modifizierten Platin-Katalysatoren zeigten jedoch eine höhere Aktivität als der mit Schwefel modifizierte Standardkatalysator VK 1.2.

**Tabelle 3 :**

| Isotherme Hochdruckhydrierung von 3,4-Dichlornitrobenzol | | | | | | | | |
|---|---|---|---|---|---|---|---|---|
| Katalysator | Beladung | | Hydrierzeit [min] | | | | | |
| | Pt [Gew.-%] | Cu [Gew.-%] | 20 | 40 | 60 | 90 | 120 | 140 |
| VK 1.2 | 1 | sulfidiert | 34,2 | 52,0 | 61,1 | 77,7 | 91,4 | 96,9 |
| K 1 | 1 | 0,1 | 45,2 | 62,5 | 81,2 | 97,8 | 100 | - |
| K 2 | 2 | 0,2 | 56,0 | 86,6 | 99,3 | 100 | - | - |
| K 3 | 3 | 0,3 | 88,1 | 99,1 | 99,3 | - | - | - |
| (Angaben in % 3,4-DCA) | | | | | | | | |

### Beispiel 4: Niederdruck Hydrierung von 3,4-Dichlornitrobenzol

Die Niederdruck-Hydrierungen wurden in einem Glasgefäß durchgeführt, in das das Reaktionsgemisch unter Atmosphärendruck eingefüllt und mit einem geringen Partialdruck von Wasserstoff-Gas beaufschlagt wurde. Die Versuchsbedingungen waren:

| | |
|---|---|
| Einwaage | 10 g 3,4-Dichlornitrobenzol (3,4-DCNB) |
| | 110 ml Ethanol |
| | 500 mg Katalysator-Trockensubstanz |
| Temperatur | T = 40° C |
| H₂-Partial-Druck | p = 10 mbar |
| Rührerdrehzahl | n = 2000 U/min |

Für die Niederdruck-Hydrierungen kamen die erfindungsgemäßen Katalsatoren K 5 bis K 11 mit unterschiedlichen Platin-Kupferverhältnissen zum Einsatz. Die Ergebnisse sind in Tabelle 4 aufgelistet. Tabelle 4 enthält neben der Reaktionszeit RZ den Wasserstoffverbrauch innerhalb dieser Zeit und die gaschromatografischen Analysen des Produktgemisches. Die Reaktionszeit RZ ist hierbei die Zeit bis zum Absinken des Wasserstoffverbrauchs auf 10 ml/min.

Die Tabelle 4 zeigt, daß die Reaktionszeit mit steigendem Kupfergehalt zunimmt, d. h. daß die Aktivität des Katalysators vermindert wird. Gleichzeitig sinkt jedoch der Anteil der Dehalogenierungsprodukte und verschwindet bei einem Platin/Kupfer-Verhältnis von 10 : 1 vollständig.

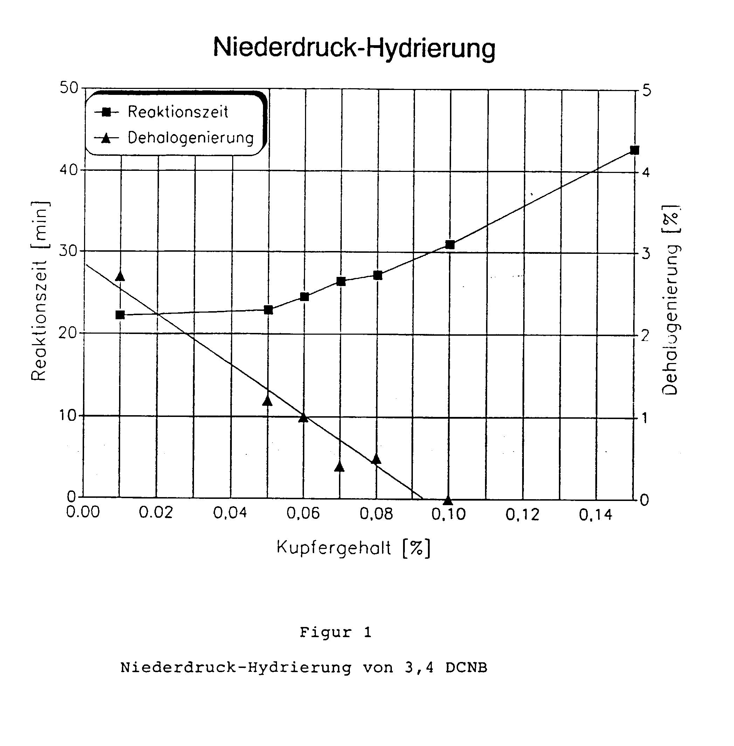

Figur 1 macht diesen Zusammenhang noch einmal grafisch deutlich und zeigt die Einzigartigkeit der erfindungsgemäß zu verwendenden Katalysatorzusammensetzung.

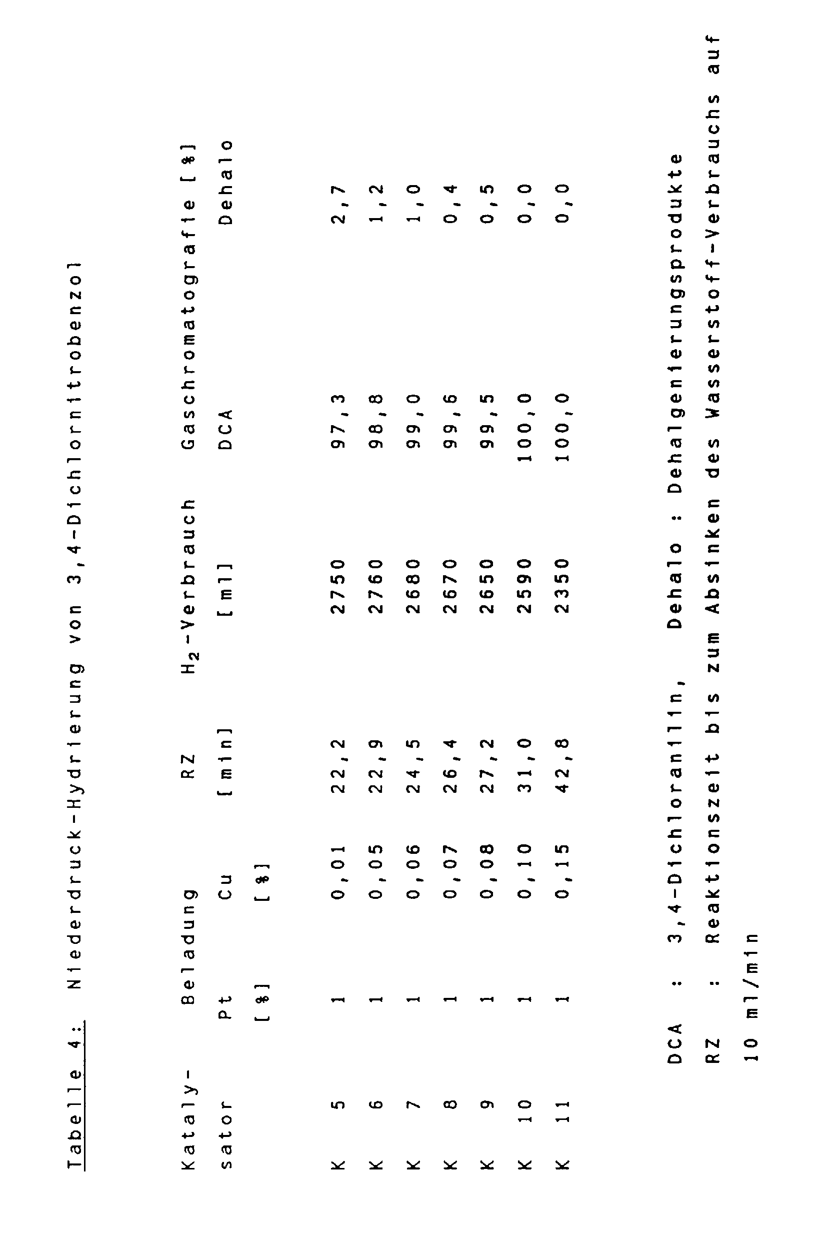

### Beispiel 5: Adiabatische Mehrzyklen-Hydrierung von 3,4-Dichlornitrobenzol

Die Hydrierungen wurden in einem 500 ml Hastelloy-Autoklaven durchgeführt. Der hierbei verwendete Autoklav unterschied sich vom Autoklaven in Beispiel 2 durch seine Rührcharakteristik und Wärmeableltungseigenschaften. Zum Einsatz kamen der Katalysator K 1 und der mit Schwefel modifizierte Vergleichskatalysator VK 1.2. Versuchsbedingungen und eingesetzte Mengen entsprachen den in Beispiel 2 angegebenen. Zur Testung der Langzeitstabilität wurden mit jeder Katalysatorprobe mehrere Chargen von 3,4-Dichlornitrobenzol hydriert. Dazu wurde der Katalysator nach jeder Hydrierung aus dem Produkt abfiltriert und für die nächste Hydrierung wiederverwendet.

Die Ergebnisse von Tabelle 5 zeigen, daß der Katalysator K1 gegenüber dem Standardkatalysator eine bessere Aktivität (kürzere Hydrierzeiten) und eine gleichmäßig hohe Selektivität besitzt. Besonders dramatisch sind die Unterschiede zwischen den beiden Katalysatortypen bei der Ausbeute. Während Katalysator K1 beim dritten Hydrierzyklus noch eine Ausbeute von 91,4 % liefert, ist die Ausbeute bei Verwendung des Standardkatalysators bei der dritten Hydrierung schon auf 54,4 % abgefallen.

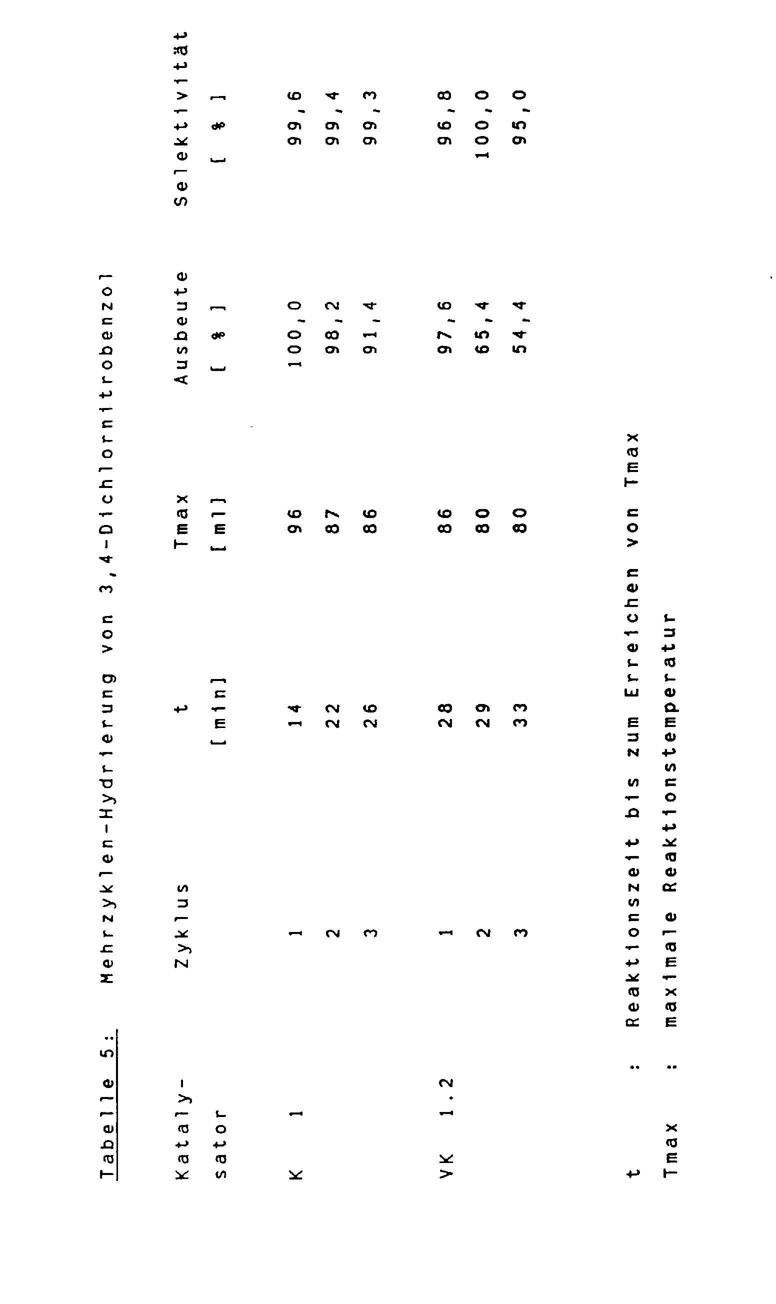

## Patentansprüche

1. Verwendung eines Katalysators aus Platin und Kupfer auf einem Träger aus Aktivkohle für die selektive Hydrierung von Halogennitroaromaten zu aromatischen Halogenaminen, welcher, bezogen auf den Aktivkohleträger, 0,1 bis 5,0, vorzugsweise 0,5 bis 3,5 Gew.-%, Platin enthält und ein Gewichtsverhältnis zwischen Platin und Kupfer von 5 : 1 bis 20 : 1, vorzugsweise 10 : 1, aufweist und erhältlich ist durch Anfertigen einer wäßrigen Suspension mit 5 bis 30 Gew.-% Aktivkohle, Hinzufügen einer wäßrigen Lösung von Platin- und Kupferverbindungen, Erwärmen der Suspension unter Rühren auf 70 bis 100°C, gleichzeitiges Niederschlagen von Platin und Kupfer auf der Aktivkohle in Form ihrer schwer löslichen Verbindungen durch Zugabe einer Base und Reduzieren dieser Verbindungen bei unveränderter Temperatur durch Zugabe eines Reduktionsmittels sowie Filtrieren und Waschen des fertigen Katalysators.

## Claims

1. Use of a catalyst of platinum and copper on a support of activated carbon for the selective hydrogenation of halonitroaromatic compounds into aromatic haloamines, which catalyst contains from 0.1 to 5.0, preferably from 0.5 to 3.5 wt.%, platinum, based on the activated carbon support, and has a weight ratio between platinum and copper of from 5 : 1 to 20 : 1, preferably 10 : 1, and is obtainable by producing an aqueous suspension with from 5 to 30 wt.% activated carbon, adding an aqueous solution of platinum and copper compounds, heating the suspension to from 70 to 100° while stirring, simultaneously depositing platinum and copper on the activated carbon in the form of their hardly soluble compounds by the addition of a base and reducing these compounds without changing the temperature by adding a reducing agent, as well as filtering and washing the final catalyst.

## Revendications

1. Utilisation d'un catalyseur à base de platine et de cuivre sur un support en charbon actif, pour l'hydrogénation sélective d'aromatiques halogénonitrés en halogénées amines aromatiques, qui rapporté au support en charbon actif, contient de 0,1 à 5,0, de préférence de 0,5 à 3,5 % en poids de platine et possède un rapport en poids entre le platine et le cuivre allant de 5:1 à 20:1, de préférence de 10:1 et est accessible par préparation d'une suspension aqueuse avec 5 à 30 % en poids de charbon actif, ajout d'une solution aqueuse de dérivés du platine et du cuivre, chauffage de la suspension sous agitation de 70 à 100°C, déposition simultanée du platine et du cuivre sur le charbon actif sous forme de leurs dérivés peu solubles par addition d'une base et réduction de ces composés à une température non modifiée par addition d'un agent de réduction ainsi que filtration et lavage du catalyseur terminé.
